# FASCICULE DE BREVET EUROPEEN

(11) **EP 1 800 669 B1**
(45) Date de publication et mention de la délivrance du brevet: **21.07.2010**
(21) Numéro de dépôt: 06291919.6
(22) Date de dépôt: 14.12.2006
(51) Int. Cl.: A61K 9/36, A61K 31/165, A61P 1/00, A61P 3/04, A61P 9/00, A61P 25/20, A61P 25/24

(54) **Composition pharmaceutique orodispersible pour administration orale, oromucosale ou sublinguale d'agomelatine**
Orodispersible pharmazeutische Zusammensetzung für die orale, oromukosale oder sublinguale Verabreichung von Agomelatin
Orodispersible pharmaceutical composition for oral, oromucosal or sublingual administration of agomelatine

(30) Priorité: 14.12.2005 FR 0512647
(43) Date de publication de la demande: 27.06.2007
(73) Titulaire: Les Laboratoires Servier, 92415 Courbevoie Cedex (FR)
(72) Inventeur: Julien, Marc, 45110 Sigloy (FR); Tharrault, François, 45000 Orleans (FR); Pean, Jean-Manuel, 45000 Orleans (FR); Wuthrich, Patrick, 45000 Orleans (FR)

(56) Documents cités:
- EP-A- 1 564 202
- FR-A- 2 834 890
- FR-A1- 2 857 263

## Description

La présente invention a pour objet une nouvelle forme pharmaceutique orodispersible solide enrobée pour l'administration par voie orale, oromucosale ou sublinguale d'agomélatine.

L'agomélatine ou N-[2-(7-méthoxy-1-naphtyl)éthyl]acétamide de formule (1) : 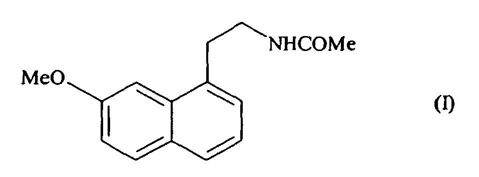 ainsi que ses hydrates, formes cristallines et sels d'addition à un acide ou à une base pharmaceutiquement acceptable, présente des propriétés pharmacologiques intéressantes : c'est un agoniste sélectif des récepteurs du système mélatoninergique et d'autre part un antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Dans tout ce qui suit, par « agomélatine » on entend l'agomélatine ainsi que ses hydrates, formes cristallines et sels d'addition à un acide ou à une base pharmaceutiquement acceptable.

L'agomélatine, peut être administré par voie orale sous forme de comprimés à libération immédiate à avaler avec un demi-verre d'eau. Ces comprimés d'agomélatine sont utiles, notamment pour le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité, et de l'ensemble des pathologies liées à un dérèglement des rythmes circadiens.

Des études pharmacocinétiques chez l'homme ont montré que la biodisponibilité de l'agomélatine par voie orale est faible par rapport à la voie parentérale et varie pour un même individu et d'un individu à l'autre.

Aussi, la biodisponibilité faible de l'agomélatine ainsi que les variations des concentrations inter et intra-individuelles ont conduit à rechercher une nouvelle formulation permettant de remédier à ces inconvénients. Une composition pharmaceutique solide orodispersible d'agomélatine décrite dans la demande de brevet EP1427724 a ainsi été mise au point contenant de l'agomélatine et des granules consistant en lactose et amidon séchés par co-atomisation et commercialisés sous l'appellation STARLAC^{®}. Cette composition pharmaceutique permet d'obtenir des comprimés ayant une très bonne aptitude à se désagréger en bouche, en respectant les critères d'orodispersibilité. Les comprimés orodispersibles permettent de délivrer le principe actif dans la cavité orale. La dissolution du principe actif dans la salive puis l'absorption par les muqueuses de la cavité orale et le passage rapide dans le sang permettent de contourner la dégradation présystémique. Ainsi, la biodisponibilité est très nettement améliorée avec des variabilités beaucoup plus faibles et une apparition rapide du principe actif dans le sang. Pour une absorption optimale à travers les muqueuses de la cavité orale, le comprimé peut être placé spécifiquement sous la langue afin de délivrer le principe actif au niveau de la muqueuse sublinguale, considérée comme la muqueuse la plus perméable de la cavité orale.
Par ailleurs, les formes pharmaceutiques orodispersibles sont reconnues pour améliorer la compliance et le confort du patient grâce à leur disparition rapide de la cavité orale sans besoin de liquide et sans difficulté pour avaler.

Cependant, il est rapidement apparu que cette formulation présentait un inconvénient provenant du principe actif utilisé, l'agomélatine, qui provoque une sensation irritante prononcée au niveau des muqueuses de la cavité orale.
Lorsque la voie sublinguale est spécifiquement ciblée, cet effet piquant est exacerbé en raison de concentrations locales élevées en agomélatine, ce qui se traduit par une très mauvaise acceptabilité par le patient.

L'utilisation de composés gustatifs répondant aux 5 descripteurs sucré, salé, acide, amer et umami n'est pas susceptible de modifier une perception douloureuse de type irritation puisque les deux systèmes chimiosensibles responsables de la transmission du goût et de l'irritation sont anatomiquement et physiologiquement différents (Franck et Rabin, Nose and Throat Journal, 1989, 68, 291-296).
Les moyens connus de l'homme de l'art pour masquer la sensation irritante provoquée par un produit destiné à se dissoudre dans la salive sont très limités : (i) utilisation d'anesthésiques locaux (WO 9915171), (ii) utilisation de molécules capables d'interagir avec les récepteurs situés sur les terminaisons nerveuses responsables des sensations irritantes (Raisinghani et Premkumar, Pain, 2005, 113, 123-133), (iii) désensibilisation des terminaisons nerveuses en assurant la libération programmée (en durée et en quantité) de produits irritants à partir de formes pharmaceutiques destinées à être maintenue dans la cavité orale pendant un temps prolongé (US 5762963).
Ces différentes approches présentent cependant des inconvénients. En effet, l'utilisation d'anesthésiques locaux ou de molécules capables d'interagir avec les récepteurs impliqués dans la sensation irritante n'est pas satisfaisante puisque ces composés comportent des propriétés pharmacologiques intrinsèques non recherchées. Par ailleurs, une stratégie reposant sur la désensibilisation n'est pas compatible avec l'obtention d'une composition pharmaceutique orodispersible (désagrégation *in vitro* inférieure à 3 minutes), c'est-à-dire qui se désagrège dans la cavité buccale en moins de 3 minutes, et de préférence en moins d'une minute.
La Demanderesse a présentement mis au point une nouvelle composition pharmaceutique permettant de pallier le problème irritant du principe actif tout en permettant d'obtenir une formulation orodispersible délivrant le principe actif par voie sublinguale ou oromucosale, et sans association avec un anesthésique local ou avec un autre composé pharmacologiquement actif.

Plus particulièrement, la présente invention concerne une composition pharmaceutique solide constituée :
* d'un noyau central ou d'une couche centrale contenant de l'agomélatine et des excipients permettant d'obtenir une formulation orodispersible,
* d'un enrobage orodispersible.

Particulièrement, la composition pharmaceutique selon l'invention est constituée d'un noyau central orodispersible contenant de l'agomélatine, et d'un enrobage orodispersible.

Une variante selon l'invention concerne une composition pharmaceutique constituée d'une couche centrale orodispersible contenant de l'agomélatine et d'un enrobage orodispersible.

Préférentiellement, le noyau central ou la couche centrale de la composition pharmaceutique solide selon l'invention contient de l'agomélatine et des excipients permettant d'obtenir une formulation orodispersible par un procédé de compression en utilisant un diluant, un lubrifiant et éventuellement un agent d'écoulement et un agent désintégrant.
Plus particulièrement, la formulation orodispersible sera obtenue avec un diluant spécifique pour orodispersible ou avec un diluant classique additionné d'un ou plusieurs agent désintégrant. Avantageusement, le diluant orodispersible utilisé sera à base de granules obtenues par co-atomisation de lactose et d'amidon et commercialisés sous l'appellation Starlac^{®}, ou sera constitué par un polyol atomisé comme par exemple le sorbitol ou le mannitol, ou par un mélange co-atomisé à base de polyols comme par exemple les excipients commercialisés sous les appellations Partek^{®} ou Pharmaburst^{®}.
D'autres diluants peuvent être utilisés, éventuellement en association avec des désintégrants, pourvu qu'ils confèrent des propriétés orodispersibles avec une dureté suffisante et une faible friabilité.
L'agent lubrifiant, l'agent d'écoulement et éventuellement l'agent désintégrant sont choisis parmi les classes respectives de ces différents excipients. Préférentiellement, l'agent lubrifiant est le stéarate de magnésium ou le sodium stéaryl fumarate.

L'enrobage orodispersible de poudre selon l'invention est préférentiellement constitué d'un diluant, d'un lubrifiant, éventuellement d'un agent d'écoulement, éventuellement d'un agent désintégrant et éventuellement d'un agent désensibilisant dilué dans l'enrobage.
Avantageusement, l'enrobage orodispersible selon l'invention contient un agent désensibilisant, comme par exemple l'acide citrique, le menthol ou l'acide aspartique.

Plus particulièrement, l'enrobage orodispersible sera obtenu avec un diluant spécifique pour orodispersible ou avec un diluant classique additionné d'un ou plusieurs agent désintégrant. Avantageusement, le diluant orodispersible utilisé sera à base de granules obtenues par co-atomisation de lactose et d'amidon et commercialisés sous l'appellation Starlac®, ou sera constitué par un polyol atomisé comme par exemple le sorbitol ou le mannitol, ou par un mélange co-atomisé à base de polyols comme par exemple les excipients commercialisés sous les appellations Partek^{®} ou Pharmaburst^{®}.
D'autres diluants peuvent être utilisés, éventuellement en association avec des désintégrants, pourvu qu'ils confèrent des propriétés orodispersibles avec une dureté suffisante et une faible friabilité.
L'agent lubrifiant, l'agent d'écoulement et éventuellement l'agent désintégrant sont choisis parmi les classes respectives de ces différents excipients. Préférentiellement, l'agent lubrifiant est le stéarate de magnésium ou le sodium stéaryl fumarate.
Parmi le ou les agents désintégrants utilisables selon l'invention, on citera ceux à base d'amidon glycolate de sodium comme par exemple le Primojel^{®} ou l'Explotab^{®}, ou à base de crospovidone comme par exemple le Kollidon^{®}CL, ou encore ceux à base de L-HPC (low-substituted hydroxypropyl cellulose).

La composition pharmaceutique solide selon l'invention est un comprimé préparé par un procédé d'enrobage par compression. Le procédé d'enrobage par compression est déjà décrit dans le cas de comprimés non orodispersibles dans le but de concevoir des formes à libération prolongée : comprimés à noyau central ou comprimés multicouches (Abdul et Poddar, Journal of Controlled Release, 2004, 97, 393-405).
Dans le cas du comprimé enrobé à couche centrale selon l'invention, la première couche d'enrobage est pré-comprimée et constitue un lit de poudre sur lequel est déposée la couche centrale. La deuxième couche d'enrobage recouvre la couche centrale avant l'étape finale de compression.
Dans le cas du comprimé à noyau central selon l'invention, l'enrobage par compression nécessite d'utiliser un noyau central suffisamment résistant pour supporter l'étape d'enrobage. Ce procédé d'enrobage n'est a priori pas recommandé dans le cas de noyaux orodispersibles démontrant généralement une résistance à l'écrasement modérée et une friabilité non négligeable. Dans le cas de la présente invention, il est possible d'enrober par compression le noyau central sans aucune difficulté, en utilisant une presse à comprimer industrielle conventionnelle adaptée à l'enrobage par compression.
La présente invention concerne donc également le procédé d'obtention du comprimé orodispersible solide à noyau central caractérisé en ce que les constituants du noyau central sont mélangés puis compressés, puis les constituants de la couche d'enrobage sont mélangés et l'enrobage effectué par compression du mélange de poudre obtenu autour du noyau.

La Demanderesse a alors trouvé que la composition pharmaceutique selon l'invention conserve son caractère orodispersible avec une très bonne aptitude à se désagréger en bouche, ce qui n'était a priori pas envisageable pour une composition pharmaceutique enrobée. En particulier, la composition pharmaceutique selon l'invention permet d'obtenir une désagrégation dans la cavité orale en moins de 3 minutes, et plus préférentiellement en moins d'une minute, sans autoriser une libération programmée capable de jouer sur les mécanismes de désensibilisation. Mais de façon surprenante, la composition pharmaceutique orodispersible selon l'invention permet d'obtenir une très bonne acceptabilité du principe actif en limitant l'aspect irritant, tout en permettant une dissolution du principe actif dans la salive et un passage rapide dans le sang.
La composition pharmaceutique selon l'invention, administrée par voie sublinguale a montré également une excellente capacité à réduire la sensation irritante provoquée par le principe actif, et donc une excellente acceptabilité par le patient. L'administration ainsi réalisée permet de limiter l'effet de premier passage hépatique, donc d'augmenter la biodisponibilité du principe actif et de diminuer la variation inter-individuelle observée avec les compositions pharmaceutiques classiques d'agomélatine destinées à l'administration entérale.

Les compositions pharmaceutiques selon l'invention sont préférentiellement caractérisées en ce qu'elles contiennent, par rapport au poids total du comprimé:
- de 0,02% à 5% en poids d'agomélatine
- de 70% à 99,88 % en poids de Starlac^{®} ou de Mannitol pour compression directe
- de 0,1 % à 3 % en poids de stéarate de magnésium.

Encore plus particulièrement, les compositions pharmaceutiques selon l'invention contiennent, par rapport au poids total du comprimé :
- de 0,02% à 5% en poids d'agomélatine
- de 70% à 99,88 % en poids de Starlac^{®} ou de Mannitol pour compression directe
- de 0,1 % à 3 % en poids de stéarate de magnésium
- de 0,5 % à 5 % en poids d'un agent désensibilisant, préférentiellement l'acide citrique, et préférentiellement entre 1 % et 3 %.

Elles contiendront éventuellement :
- un agent lubrifiant autre que le stéarate de magnésium comme par exemple 0,1 % à 3% en poids de stéaryl-fumarate de sodium, préférentiellement de 0,5 % à 1,5% ;
- un agent d'écoulement comme la silice colloïdale par exemple, de 0,1 % à 3 % en poids, préférentiellement de 0,2 % à 1 % ;
- 0,01 % à 5 % en poids d'un ou plusieurs édulcorants, préférentiellement de 0,1 % à 1 %.

En outre, les compositions pharmaceutiques selon l'invention pourront contenir des composés aromatisants, des substances colorantes et un ou plusieurs édulcorants comme le saccharose, l'aspartam, l'acésulfam ou le sucralose.

La posologie utile est adaptable selon la nature et la sévérité de l'affection, la voie d'administration ainsi que l'âge et le poids du patient. Cette posologie varie de 0,1 mg à 1 g d'agomélatine par jour en une ou plusieurs prises.

Les exemples suivants illustrent l'invention mais ne la limitent en aucune façon :

### EXEMPLE 1

### Formulation : Comprimé terminé à 320 mg

| ***Constituants*** | ***Quantité (mg)*** |
|---|---|
| *Noyau central* | |
| Agomélatine | 1 |
| Starlac® | 67,65 |
| Sodium stéaryl fumarate | 1,35 |
| *Enrobage* | |
| Acide citrique anhydre broyé | 7,5 |
| Starlac® | 238,75 |
| Acesulfam potassium | 2,5 |
| Sodium stéaryl fumarate | 1,25 |

### EXEMPLE 2

### Formulation : Comprimé terminé à 350 mg

| ***Constituants*** | ***Quantité (mg)*** |
|---|---|
| *Noyau central* | |
| Agomélatine | 1 |
| Starlac® | 48,75 |
| Stéarate de magnésium | 0,25 |
| *Enrobage* | |
| Acide citrique anhydre broyé | 10 |
| Starlac® | 282 |
| Aspartam | 3 |
| Acesulfam potassium | 3 |
| Stéarate de magnésium | 2 |

Le noyau central est préparé par mélange des constituants suivi d'une compression directe. La dureté des noyaux des exemples 1 et 2 est environ égale à 15 newtons avec une friabilité inférieure à 1%. Les constituants de la couche d'enrobage sont mélangés puis l'enrobage est effectué par compression du mélange de poudre obtenu autour des noyaux.
Les comprimés enrobés des exemples 1 et 2 ont une dureté cible de 40 newtons et une friabilité autour de 1%. Les temps de désagrégation *in vitro* sont inférieurs à 3 minutes (Pharmacopée Européenne).

### EXEMPLE 3

### Formulation : Comprimé terminé à 320 mg

| ***Constituants*** | ***Quantité (mg)*** |
|---|---|
| *Noyau central* | |
| Agomélatine | 1 |
| Mannitol pour compression directe | 68,3 |
| Stéarate de magnésium | 0,7 |
| *Enrobage* | |
| Acide citrique anhydre broyé | 7,5 |
| Mannitol pour compression directe | 235 |
| Acesulfam potassium | 2,5 |
| Aspartam | 2,5 |
| Stéarate de magnésium | 2,5 |

### EXEMPLE 4

### Formulation : Comprimé terminé à 320 mg

| ***Constituants*** | ***Quantité (mg)*** |
|---|---|
| *Couche centrale* | |
| Agomélatine | 2 |
| Starlac® | 67,65 |
| Stéarate de magnésium | 0,35 |
| *Enrobage (2 couches externes de 125 mg chacune)* | |
| Acide citrique anhydre broyé | 7,5 |
| Starlac® | 238,75 |
| Sucralose | 2,5 |
| Stéarate de magnésium | 1,25 |

Les constituants des différentes couches sont mélangés à l'aide d'un mélangeur par retournement puis les mélanges de poudres obtenus sont disposés dans les trémies d'alimentation d'une machine à comprimer comportant plusieurs stations de compression. La première couche d'enrobage (125 mg) est pré-comprimée et constitue un lit de poudre sur lequel est déposée la couche centrale. La deuxième couche d'enrobage recouvre la couche centrale avant l'étape finale de compression. Les comprimés enrobés obtenus ont une dureté cible de 40 newtons et une friabilité autour de 1%. Les temps de désagrégation *in vitro* sont inférieurs à 3 minutes (Pharmacopée Européenne).

### EXEMPLE 5

### Acceptabilité

Une étude d'acceptabilité chez 12 volontaires sains de sexe masculin a été réalisée en administrant en prise unique soit une composition orodispersible conventionnelle contenant 3% d'acide citrique et 1 mg d'agomélatine dilués dans la masse (comprimé orodispersible non enrobé terminé à 320 mg), soit un comprimé orodispersible à noyau central contenant 1 mg d'agomélatine dans le noyau, 3% d'acide citrique dans l'enrobage et terminé à 320 mg. L'étude réalisée a montré que 67% des volontaires ont bien ou modérément apprécié le comprimé orodispersible enrobé contre 50% seulement pour le comprimé orodispersible conventionnel.

## Revendications

1. Composition pharmaceutique solide orodispersible enrobée d'agomélatine **caractérisée en ce qu'**elle comprend :
- un noyau central ou une couche centrale contenant de l'agomélatine et des excipients permettant d'obtenir une formulation orodispersible,
- un enrobage orodispersible.

2. Composition pharmaceutique selon la revendication 1 **caractérisée en ce que** l'agomélatine est obtenu sous la forme cristalline II.

3. Composition pharmaceutique selon la revendication 1 **caractérisée en ce qu'**elle contient un noyau central.

4. Composition pharmaceutique selon la revendication 1 **caractérisée en ce qu'**elle contient une couche centrale.

5. Composition pharmaceutique selon la revendication 1 **caractérisée en ce que** le noyau ou la couche centrale contient un diluant conférant une propriété orodispersible.

6. Composition pharmaceutique selon la revendication 5 **caractérisée en ce que** le diluant utilisé dans le noyau central est à base de granules obtenues par co-atomisation de lactose et d'amidon.

7. Composition pharmaceutique selon la revendication 1 **caractérisée en ce que** le noyau ou la couche centrale contient un diluant et un agent désintégrant.

8. Composition pharmaceutique selon la revendication 1 **caractérisée en ce que** l'enrobage orodispersible contient un diluant conférant une propriété orodispersible.

9. Composition pharmaceutique selon la revendication 8 **caractérisée en ce que** le diluant utilisé dans l'enrobage est à base de granules obtenues par co-atomisation de lactose et d'amidon.

10. Composition pharmaceutique selon la revendication 1 **caractérisée en ce que** l'enrobage orodispersible contient un agent désensibilisant.

11. Composition pharmaceutique selon la revendication 10 **caractérisée en ce que** l'agent désensibilisant utilisé est l'acide citrique.

12. Composition pharmaceutique selon la revendication 1 **caractérisée en ce qu'**elle comprend, par rapport au poids total de la composition :
- de 0,02% à 5% en poids d'agomélatine
- de 70% à 99,88 % en poids de Starlac^{®} ou de Mannitol pour compression directe
- de 0,1 % à 3 % en poids de stéarate de magnésium.

13. Composition pharmaceutique selon la revendication 1 **caractérisée en ce qu'**elle comprend, par rapport au poids total de la composition :
- de 0,02% à 5% en poids d'agomélatine
- de 70% à 99,88 % en poids de Starlac^{®} ou de Mannitol pour compression directe
- de 0,1 % à 3 % en poids de stéarate de magnésium
- de 0,5 % à 5 % en poids d'un agent désensibilisant.

14. Composition pharmaceutique selon la revendication 1 **caractérisée en ce qu'**elle comprend un ou plusieurs lubrifiants, et également un agent d'écoulement, et un ou plusieurs édulcorants.

15. Composition pharmaceutique selon la revendication 1 **caractérisée en ce qu'**elle se présente sous forme de comprimé à noyau central.

16. Composition pharmaceutique selon la revendication 1 **caractérisée en ce qu'**elle se présente sous forme de comprimé tricouche.

17. Procédé d'obtention de la composition pharmaceutique selon la revendication 15 **caractérisé en ce que** les constituants du noyau sont mélangés puis compressés par compression directe, puis les constituants de la couche d'enrobage sont mélangés et l'enrobage effectué par compression du mélange de poudre obtenu autour des noyaux.

18. Utilisation de granules consistant en lactose et amidon séchés par co-atomisation et d'acide citrique dans la fabrication de l'enrobage des compositions solides orodispersibles à noyau central ou couche centrale d'agomélatine selon la revendication 1 se délitant en bouche en moins de trois minutes et de préférence en moins d'une minute.

19. Composition pharmaceutique solide orodispersible à noyau central ou couche centrale d'agomélatine, selon l'une des revendications 1 à 16, utile pour le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité et de l'ensemble des pathologies liées à un dérèglement des rythmes circadiens.

## Claims

1. Coated solid orodispersible pharmaceutical composition of agomelatine, **characterised in that** it comprises:
- a central core or a central layer comprising agomelatine and excipients allowing an orodispersible formulation to be obtained,
- an orodispersible coating.

2. Pharmaceutical composition according to claim 1, **characterised in that** the agomelatine is obtained in crystalline form II.

3. Pharmaceutical composition according to claim 1, **characterised in that** it contains a central core.

4. Pharmaceutical composition according to claim 1, **characterised in that** it contains a central layer.

5. Pharmaceutical composition according to claim 1, **characterised in that** the central core or layer comprises a diluent imparting an orodispersible property.

6. Pharmaceutical composition according to claim 5, **characterised in that** the diluent used in the central core is based on granules obtained by co-atomisation of lactose and starch.

7. Pharmaceutical composition according to claim 1, **characterised in that** the central core or layer comprises a diluent and a disintegrating agent.

8. Pharmaceutical composition according to claim 1, **characterised in that** the orodispersible coating comprises a diluent imparting an orodispersible property.

9. Pharmaceutical composition according to claim 8, **characterised in that** the diluent used in the coating is based on granules obtained by co-atomisation of lactose and starch.

10. Pharmaceutical composition according to claim 1, **characterised in that** the orodispersible coating comprises a desensitising agent.

11. Pharmaceutical composition according to claim 10, **characterised in that** the desensitising agent used is citric acid.

12. Pharmaceutical composition according to claim 1, **characterised in that** it comprises, in relation to the total weight of the composition:
- from 0.02% to 5% by weight of agomelatine
- from 70% to 99.88% by weight of Starlac^{®} or mannitol for direct compression
- from 0.1 % to 3 % by weight of magnesium stearate.

13. Pharmaceutical composition according to claim 1, **characterised in that** it comprises, in relation to the total weight of the composition:
- from 0.02% to 5% by weight of agomelatine
- from 70% to 99.88% by weight of Starlac^{®} or mannitol for direct compression
- from 0.1 % to 3% by weight of magnesium stearate
- from 0.5% to 5% by weight of a desensitising agent.

14. Pharmaceutical composition according to claim 1, **characterised in that** it contains one or more lubricants, and also a flow agent, and one or more sweetening agents.

15. Pharmaceutical composition according to claim 1, **characterised in that** it is presented in the form of a tablet having a central core.

16. Pharmaceutical composition according to claim 1, **characterised in that** it is presented in the form of a three-layer tablet.

17. Method of obtaining the pharmaceutical composition according to claim 15, **characterised in that** the constituents of the core are mixed and then compressed by direct compression, and then the constituents of the coating layer are mixed and the coating is carried out by compression of the resulting powder mixture around the cores.

18. Use of granules consisting of lactose and starch dried by co-atomisation and of citric acid in the manufacture of a coating for solid orodispersible compositions having a central core or central layer of agomelatine according to claim 1 that disintegrate in the mouth in less than three minutes and preferably in less than one minute.

19. Solid orodispersible pharmaceutical composition having a central core or central layer of agomelatine according to one of claims 1 to 16 for use in the treatment of major depression, seasonal affective disorder, sleep disorders, cardiovascular pathologies, pathologies of the digestive system, conditions of insomnia and fatigue resulting from jetlag, appetite disorders and obesity, and all pathologies associated with deregulation of circadian rhythms.

## Patentansprüche

1. Feste, in der Mundhöhle dispergierbare pharmazeutische Zusammensetzung mit umhülltem Agomelatin, **dadurch gekennzeichnet, dass** sie:
- einen zentralen Kern oder eine zentrale Schicht, die Agomelatin und Hilfsstoffe zum Erhalt einer in der Mundhöhle dispergierbaren Formulierung und
- eine in der Mundhöhle dispergierbare Umhüllung
umfasst.

2. Pharmazeutische Zusammensetzung nach Anspruch 1, **dadurch gekennzeichnet, dass** Agomelatin in der Kristallform II enthalten ist.

3. Pharmazeutische Zusammensetzung nach Anspruch 1, **dadurch gekennzeichnet, dass** sie einen zentralen Kern enthält.

4. Pharmazeutische Zusammensetzung nach Anspruch 1, **dadurch gekennzeichnet, dass** sie eine zentrale Schicht enthält.

5. Pharmazeutische Zusammensetzung nach Anspruch 1, **dadurch gekennzeichnet, dass** der zentrale Kern oder die zentrale Schicht ein Verdünnungsmittel enthält, welches die Fähigkeit der Dispergierbarkeit in der Mundhöhle verleiht.

6. Pharmazeutische Zusammensetzung nach Anspruch 5, **dadurch gekennzeichnet, dass** das in dem zentralen Kern verwendete Verdünnungsmittel auf der Grundlage von Körnchen vorliegt, die durch gleichzeitige Zerstäubung von Lactose und Stärke erhalten worden sind.

7. Pharmazeutische Zusammensetzung nach Anspruch 1, **dadurch gekennzeichnet, dass** der zentrale Kern oder die zentrale Schicht ein Verdünnungsmittel und ein Sprengmittel enthält.

8. Pharmazeutische Zusammensetzung nach Anspruch 1, **dadurch gekennzeichnet, dass** die in der Mundhöhle dispergierbare Umhüllung ein Verdünnungsmittel enthält, welches die Fähigkeit der Dispergierbarkeit in der Mundhöhle verleiht.

9. Pharmazeutische Zusammensetzung nach Anspruch 8, **dadurch gekennzeichnet, dass** das in der Umhüllung verwendete Verdünnungsmittel auf der Grundlage von Körnchen vorliegt, die durch gemeinsames Zerstäuben von Lactose und Stärke erhalten worden sind.

10. Pharmazeutische Zusammensetzung nach Anspruch 1, **dadurch gekennzeichnet, dass** die in der Mundhöhle dispergierbare Umhüllung ein Desensibilisierungsmittel enthält.

11. Pharmazeutische Zusammensetzung nach Anspruch 10, **dadurch gekennzeichnet, dass** das verwendete Desensibilisierungsmittel Citronensäure ist.

12. Pharmazeutische Zusammensetzung nach Anspruch 1, **dadurch gekennzeichnet, dass** sie, bezogen auf das Gesamtgewicht der Zusammensetzung, enthält:
- 0,02 % bis 5 Gew.-% Agomelatin,
- 70 % bis 99,88 Gew.-% Starlac^{®} oder Mannitol für das direkte Verpressen,
- 0,1 % bis 3 Gew.-% Magnesiumstearat.

13. Pharmazeutische Zusammensetzung nach Anspruch 1, **dadurch gekennzeichnet, dass** sie, bezogen auf das Gesamtgewicht der Zusammensetzung, enthält:
- 0,02 % bis 5 Gew.-% Agomelatin,
- 70 % bis 99,88 Gew.-% Starlac^{®} oder Mannitol für das direkte Verpressen,
- 0,1 % bis 3 Gew.-% Magnesiumstearat,
- 0,5 % bis 5 Gew.-% eines Desensibilisierungsmittels.

14. Pharmazeutische Zusammensetzung nach Anspruch 1, **dadurch gekennzeichnet, dass** sie ein oder mehrere Gleitmittel und ebenfalls ein Fließmittel und ein oder mehrere Süßungsmittel enthält.

15. Pharmazeutische Zusammensetzung nach Anspruch 1, **dadurch gekennzeichnet, dass** sie in Form einer Tablette mit zentralem Kern vorliegt.

16. Pharmazeutische Zusammensetzung nach Anspruch 1, **dadurch gekennzeichnet, dass** sie in Form einer dreischichtigen Tablette vorliegt.

17. Verfahren zur Herstellung der pharmazeutischen Zusammensetzung nach Anspruch 15, **dadurch gekennzeichnet, dass** man die Bestandteile für den Kern vermischt und durch direktes Verpressen verpresst, dann die Bestandteile für die Umhüllungsschicht vermischt und das Umhüllen durch Verpressen der erhaltenen Pulvermischung um die Kerne herum bewirkt.

18. Verwendung von Körnchen, die durch gemeinsames Zerstäuben getrocknete Lactose und Stärke und Citronensäure, enthalten bei der Herstellung der Umhüllung von in festen, in der Mundhöhle dispergierbaren Zusammensetzungen mit zentralem Kern oder zentraler Schicht aus Agomelatin nach Anspruch 1, die sich in weniger als drei Minuten und vorzugsweise weniger als einer Minute im Mundraum zersetzen.

19. In der Mundhöhle dispergierbare pharmazeutische Zusammensetzung mit zentralem Kern oder zentraler Schicht aus Agomelatin nach einem der Ansprüche 1 bis 16, nützlich für die Behandlung von Major-Depression, saisonal bedingten Depressionen, Schlafstörungen, kardiovaskulären Erkrankungen, Erkrankungen des Verdauungssystems, Schlaflosigkeit und Müdigkeit als Folge von Zeitverschiebungen, Appetitstörungen und Fettsucht und die Gesamtheit von Erkrankungen, die mit einer Deregulierung der 24-Stunden-Rhythmen verknüpft sind.
